Europäisches Patentamt

European Patent Office

Office européen des brevets

(11) Numéro de publication : **0 484 430 B1**

(12) **FASCICULE DE BREVET EUROPEEN**

(45) Date de publication du fascicule du brevet :
01.12.93 Bulletin 93/48

(21) Numéro de dépôt : **90912241.8**

(22) Date de dépôt : **24.07.90**

(86) Numéro de dépôt international :
**PCT/FR90/00560**

(87) Numéro de publication internationale :
**WO 91/02265 21.02.91 Gazette 91/05**

(51) Int. Cl.⁵ : **G01T 1/164**

(54) **DISPOSITIF A SCINTILLATION UTILISABLE POUR MESURER L'ATTENUATION PAR TOMOGRAPHIE DE TRANSMISSION.**

(30) Priorité : **28.07.89 FR 8910225**

(43) Date de publication de la demande :
**13.05.92 Bulletin 92/20**

(45) Mention de la délivrance du brevet :
**01.12.93 Bulletin 93/48**

(84) Etats contractants désignés :
**AT DE ES FR GB IT NL**

(56) Documents cités :
**EP-A- 0 092 437
US-A- 4 788 429
The Journal of Nuclear Medicine, Vol. 27, No. 5, May 1986, (New York, N.Y., US), J.A. Malko et al: "SPECT liver imaging using an iterative attenuation correction algorithm and an external flood source", pages 701-705**

(73) Titulaire : **SOPHA MEDICAL
9, Place de la Madeleine
F-75008 Paris (FR)**
Titulaire : **COMMISSARIAT A L'ENERGIE ATOMIQUE
31-33, rue de la Fédération
F-75015 Paris (FR)**

(72) Inventeur : **BOURGUIGNON, Michel
20, rue Paul-Couderc
F-92390 Sceaux (FR)**
Inventeur : **VASSILIOU, Marie-Michèle
10, rue Poinsot
F-75014 Paris (FR)**
Inventeur : **de la BARRE, François
10, place du Théâtre
F-92310 Sèvres (FR)**

(74) Mandataire : **Schmit, Christian Norbert Marie
Cabinet Ballot-Schmit 7, rue Le Sueur
F-75116 Paris (FR)**

EP 0 484 430 B1

## Description

La présente invention a pour objet un dispositif à scintillation utilisable pour mesurer l'atténuation par tomographie de transmission. Elle trouve son application plus particulièrement dans le domaine médical où, en association avec une gamma caméra utilisée classiquement, elle permet d'affiner la connaissance des structures internes révélées par un examen de tomographie en émission exécuté au moyen de cette gamma caméra.

Un examen de tomographie en émission exécuté au moyen d'une gamma caméra comporte l'injection, dans un corps à examiner, d'un marqueur radioactif. Dans le domaine médical, le marqueur, par exemple du Technetium, est injecté, sous forme d'une dilution, dans le sang du patient. En circulant dans le corps de ce dernier,le marqueur radioactif produit un phénomène radioactif mesurable. Le phénomène radioactif comporte essentiellement l'émission de rayons gamma.

Pour mesurer ce phénomène radioactif, on utilise une gamma caméra, ou caméra à scintillation munie d'un cristal scintillateur plan. Une gamma caméra comporte essentiellement un statif pour supporter un détecteur surmonté du cristal scintillateur. Une gamma caméra comporte aussi une console de commande et de traitement. Le détecteur comporte un arrangement de tubes photomultiplicateurs pour produire des signaux électriques correspondant à des scintillations détectées.Le scintillateur absorbe le rayonnement radioactif gamma par effet photoélectrique. Il émet une scintillation lumineuse détectée en aval par le réseau des tubes photomultiplicateurs du détecteur. Les tubes sont associés à des moyens de calcul. Ceux-ci permettent de déterminer les coordonnées d'un lieu d'interaction des rayonnements gamma dans le scintillateur.

Compte-tenu de ce que les émissions radioactives dans le corps sont omnidirectionnelles, cette localisation ne peut être menée à bien qu'en interposant entre le corps et le scintillateur un collimateur. Ce collimateur ne laisse passer que les rayonnements radioactifs dans une direction choisie.

Avec de tels examens, on est capable de produire des images en projection. Si on fait tourner la gamma caméra autour du corps du patient pendant que le phénomène radioactif se produit (pendant une durée d'examen d'environ une demi heure), on est susceptible d'acquérir un certain nombre de projections, de types parallèles, avec lesquelles selon des procédés de tomographie de type connu, on sait reconstruire des images de coupe. Les projections sont de type parallèle parce que le collimateur ne laisse passer les rayons que dans une seule direction perpendiculaire à son plan.

Le mode d'acquisition ainsi rappelé présente cependant un inconvénient : les rayons gamma émis par les structures internes du corps doivent, avant d'aller exciter le scintillateur, traverser d'autres régions de ce corps et ils en subissent une atténuation correspondante. Celle-ci perturbe l'acquisition des données et l'exactitude et la précision des images reconstruites.

De nombreuses tentatives ont été faites pour prendre en compte cette atténuation sans la mesurer vraiment. Cependant, les méthodes ainsi proposées se sont révélées peu fructueuses, et à ce jour la mesure réelle de l'atténuation par tomographie de transmission est la seule solution qui paraisse envisageable.

On pourrait par exemple envisager de mesurer l'atténuation radiologique en utilisant les mesures de tomodensitométrie proposées par les tomodensitomètres modernes utilisant des rayons X.

Cette technique présente cependant deux inconvénients. Premièrement, il faut déplacer le patient d'une machine à une autre. On n'est jamais assuré qu'il reprendra dans la deuxième machine la même position que celle qu'il avait dans la première. Les mesures deviennent difficilement comparables ou transposables. D'autre part, les énergies du rayonnement mis en oeuvre dans un cas et dans l'autre sont différentes. Il s'agit de rayonnements X en tomodensitométrie et il s'agit de rayonnements gamma dans le dispositif à scintillation, et les coefficients d'atténuation mesurés sont tous dépendants de l'énergie du rayonnement absorbé.

Une méthode de mesure de l'atténuation par tomographie de transmission; utilisant une gamma caméra rotative munie de son collimateur et une large source plane radioactive extérieure, a été testée avec un certain succès.

Cependant, plusieurs écueils sérieux limitent l'utilisation pratique de cette méthode. D'une part, la durée d'acquisition supplémentaire (acquisition pour la mesure de l'atténuation par transmission) est trop longue. En effet, elle dure environ 30 minutes. Par ailleurs, une source plane radioactive est difficile à préparer en ce sens qu'on n'est jamais assuré de l'homogénéité de l'émission des différentes parties de la source. Elle est également difficile à manipuler du fait de son poids. Enfin et surtout, elle provoque une irradiation permanente inadmissible des personnes qui sont appelés à la mettre en oeuvre. Une telle technique a par exemple été décrite par Malko J.A. & Al. dans un article intitulé : "SPECT liver imaging using an iterative attenuation correction algorithm and an external flood source", publié dans Journal of Nuclear Medecine 27:701-705, 1986.

Autres méthodes ont proposé d'associer une source gamma ponctuelle à une gamma caméra sans collimateur. Dans US-A-4 788 429 (Wilson) le collimateur est remplacé par une grille radiographique pour remédier à la dispersion du rayonnement gamma. Dans l'article de Deconinck F. & Al., intitulé : "Computerized transmission densitography and tomography with a gamma camera" au Colloque de l'INSERM, INSERM 1979, volume 88, pages 245 - 256, une source ponctuelle est située à 2 mètres d'une gamma caméra dans un plan horizontal. Le patient est approché sur une chaise tournante entre cette source et cette gamma caméra. La chaise tourne autour d'un axe vertical. La distance de 2 mètres est une distance minimum pour permettre de considérer le rayonnement gamma émis par la source ponctuelle, comme un rayonnement parallèle à l'endroit du corps irradié. L'acquisition des différentes projections est faite en faisant tourner la chaise.

Cette dernière technique présente deux inconvénients. D'une part, l'éloignement de la source est tel qu'il devient inenvisageable de produire une structure suffisamment rigide qui permettrait de tenir en correspondance la gamma caméra, lourde, et la source lorsque cet ensemble tournerait autour du corps, sans avoir à souffrir des vibrations. De plus, les volumes nécessaires pour les salles d'examen médicaux sortiraient des normes. D'autre part, cette impossibilité impose que c'est le patient qui doit être mis en mouvement par rapport à la caméra, ce qui, dans certains cas, est impossible : en particulier quand les patients sont faibles physiquement et ne peuvent pas supporter la position verticale ou assise. Il faut prendre en compte en effet un tassement du patient sur lui-même au cours du temps. Ceci provoque l'interpénétration des coupes acquises et fausse en conséquence la reconstruction.

L'inconvénient qu'il y a à rapprocher la source de la gamma caméra pour constituer ainsi une structure manipulable autour du patient se situe dans le fait qu'on perd le caractère parallèle du rayonnement qui interdit par la suite l'utilisation des algorithmes connus de reconstruction des images d'atténuation acquises.

L'invention a pour objet de remédier à ces inconvénients en utilisant quand même une source ponctuelle disposée en correspondance et à proximité de la gamma caméra. En pratique la proximité est inférieure à 1 mètre : dans un exemple préféré elle est de 70 cm.

Pour résoudre alors les problèmes de géométrie cônique provoqués par cette proximité, on exécute une pondération correspondant à un réarrangement de l'ensemble des projections côniques ainsi obtenues en un ensemble de projections parallèles, et une log normalisation par l'intensité de la source. Dans ces conditions, on peut disposer d'une source ponctuelle proche dont l'efficacité est encore augmentée du fait de sa proximité plus grande du corps. On n'utilise alors pas, dans ce cas, de collimateur, ce qui rend la gamma caméra 300 à 500 fois plus sensible que si elle en possédait un. Dans ces conditions, on arrive à réduire les temps d'acquisition pour la mesure de l'atténuation en transmission à 1 minute environ : le temps nécessaire pour acquérir 64 projections pendant une durée d'1 seconde pour chaque projection.

Le matériel utilisable devient alors un matériel standard : le même que celui qui était utilisé pour la tomographie d'émission (celle qui concerne la révélation de la présence des marqueurs dans le corps). Dans l'invention, compte-tenu de ce qu'on utilise un matériel standard, on a judicieusement remplacé le collimateur, qui a été enlevé, par un cadre plombé de même poids obturé au besoin par une plaque de plastique transparent aux rayonnements gamma. Ceci permet de conserver l'équilibre de la gamma caméra conventionnelle utilisée. Si la gamma caméra est ronde, le cadre est rond, si elle est rectangulaire, le cadre est rectangulaire. Le cadre plombé joue surtout le rôle d'un obturateur de champ réduisant la surface de détection à la zone utile et permettant d'utiliser alors le taux de comptage de la gamma caméra à la révélation des phénomènes effectifs.

Avec le dispositif de l'invention, on obtient une bonnne résolution image liée à la qualité de résolution intrinsèque du détecteur sans collimation. Ceci est dû aussi à l'effet grossissant de la géométrie cônique liée à la proximité choisie de la source de rayons gamma. Aussi, malgré les aberrations liées à cette géométrie cônique à trois dimensions, on obtient un résultat meilleur.

En outre, on a mis à profit l'utilisation d'une source ponctuelle pour réaliser une source radioactive dont l'emploi serait d'une part aisé et sans danger pour les manipulateurs, et dont la mise en oeuvre ne présente pas les autres problèmes rencontrés dans l'état de la technique. En effet, les seules sources envisageables sont des sources liquides. Le fait de déplacer la source impose de devoir gérer la position d'une bulle d'air qui se déplace selon que la source est placée en dessous ou au-dessus de la gamma caméra, selon qu'elle est retournée sur elle-même ou non. Dans l'invention, la source ponctuelle est réalisée au moyen d'une seringue remplie puis partiellement vidée, en en plongeant l'extrémité dans un bocal, et en exprimant la bulle d'air par réinjection dans le bocal. De cette manière, la seringue réalisée ne comporte plus de bulle d'air. La position du centre de gravité de la source radioactive est alors maintenue fixe par rapport à la seringue quelle que soit l'orientation de cette seringue (orientée vers le haut ou vers le bas).

L'invention a donc pour objet un dispositif à scintillation comportant une gamma caméra, utilisable pour mesurer l'atténuation par transmission, au travers d'un corps à examiner, d'un rayonnement gamma émis par une source ponctuelle radioactive maintenue en correspondance d'un détecteur de ladite gamma caméra, ledit détecteur étant démuni d'un collimateur et- étant placé d'un premier coté du corps, opposé par rapport à un autre coté du corps où est placée la source ponctuelle, et ledit corps étant déplacé par un mouvement de rotation relatif par rapport à l'ensemble formé par ladite source ponctuelle et ledit détecteur, caractérisé en ce que ladite gamma caméra est montée sur une structure mobile, cette structure étant susceptible de faire tourner le détecteur de la gamma caméra dans un plan vertical autour du corps disposé en position allongée sur un support horizontal, en ce que cette structure entraîne également la source ponctuelle de rayonnement gamma, et des moyens pour pondérer l'atténuation mesurée en fonction de l'éloignement source détecteur et en fonction du caractère plan du détecteur.

L'invention sera mieux comprise à la lecture de la description qui suit et à l'examen des figures qui l'accompagnent. Celles-ci ne sont données qu'à titre indicatif et nullement limitatif de l'invention. Les figures montrent :

- Figure 1 : la représentation schématique d'une gamma caméra selon l'invention ;
- Figure 2 : le mode de réalisation d'une source radioactive selon l'invention ;
- Figure 3 : un diaphragme pour limiter le champ de la gamma caméra à une surface inférieure à la surface projetée du corps.
- Figure 4 : une représentation schématique en coupe de la pondération effectuée.

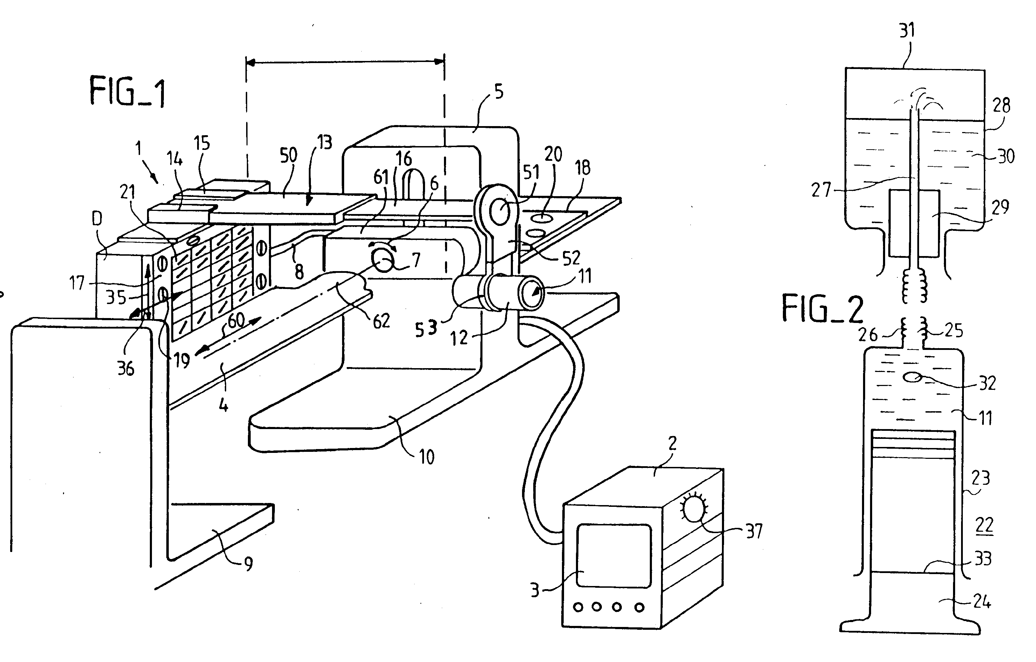

La figure 1 montre un dispositif à scintillation selon l'invention. Celui-ci comporte principalement une gamma caméra 1 munie de son scintillateur non représenté, d'un réseau 21 de tubes photomultiplicateurs de son détecteur D et d'un ensemble de traitement 2 et de visualisation 3 d'images d'émission acquises par la gamma caméra 1. Le détecteur de la gamma caméra est susceptible de tourner autour du corps d'un patient non représenté placé sur un panneau porte-patient 4. Le détecteur de la gamma caméra dans ce but est maintenu par un bâti 5 par une structure 8 en forme de potence. La structure 8 peut tourner, selon le sens de la flèche 6 autour d'un axe 7 horizontal. Ceci permet au détecteur de la gamma caméra 1 de tourner dans un plan vertical autour de l'axe 7 horizontal. La potence 8 est fixée d'une part au détecteur de la gamma caméra 1 et d'autre part peut tourner, animée par un moteur non représenté, autour de l'axe 7.

Le panneau 4 et le bâti 5 sont maintenus par des socles respectivement 9 et 10 l'un en face de l'autre. Les socles peuvent être mobiles l'un par rapport à l'autre pour déplacer le corps devant le détecteur.

Le dispositif à scintillation de l'invention comporte également une source radioactive 11 considérée comme ponctuelle et située à l'intérieur d'une enceinte de protection 12. L'enceinte de protection 12 est destinée à protéger les manipulateurs des radiations radioactives émises dans tout l'espace par la source 11. L'enceinte 12, par exemple en plomb, est donc munie d'un côté faisant face au détecteur de la gamma caméra 1 d'un orifice par lequel peuvent se propager les rayons émis par la source 11. La source 11 est représentée sur la figure 2. L'enceinte 12 peut comporter, du côté opposé à son orifice, une ouverture obturable qui permet l'introduction de la source radioactive à l'intérieur.

L'enceinte 12 est maintenue solidaire de la gamma caméra 1 par bras potencé 13. Le bras 13 est fixé au détecteur D par engagement dans un jeu de coulisseaux 14 et 15 fixés à ce détecteur. Le bras 13 s'élève alors sensiblement perpendiculairement au plan du détecteur D. Le bras 13 est télescopique et comporte un avant-bras 16 qui peut pénétrer à l'intérieur de la base 50 du bras 13. L'avant-bras 16 est terminé en prolongement par un pivot 51 sur lequel est monté, libre en rotation, perpendiculairement à l'axe du pivot, une flèche télescopique 52. La flèche 52, à son extrémité opposée au pivot, porte une couronne 53 destinée à recevoir l'enceinte 12 en coulissement intérieur. Le caractère télescopique du bras 13 et de la flèche 52, ainsi que la rotation autour du pivot 51 et le coulissement de l'enceinte 12 permettent de donner à la source n'importe quelle position choisie dans l'espace par rapport au détecteur D.

Le détecteur D est surmonté d'un cadre 17. Le cadre 17, par exemple en plomb, a de préférence les mêmes dimensions extérieures qu'un collimateur 18 de la gamma caméra qui a été provisoirement enlevé. Il a la même forme : si le collimateur est rond, le cadre est rond, si le collimateur est rectangulaire, le cadre est rectangulaire. Le cadre 17 possède également des dispositifs de fixation, par exemple par des vis 19, qui occupent exactement les mêmes places et qui s'imbriquent exactement de la même manière que les dispositifs de fixation 20 du collimateur 18 sur la gamma caméra 1. Le cadre 17 est placé à la périphérie du réseau 21 des tubes photomultiplicateurs du détecteur de la gamma caméra 1. Ces tubes photomultiplicateurs sont montrés, ici symboliquement, parce qu'en fait ils ne sont pas visibles (ils sont masqués par la présence du scintillateur toujours nécessaire). Le réseau de ces tubes peut être rond, hexagonal ou même rectangulaire. Les tubes eux-mêmes peuvent avoir une section ronde, hexagonale ou carrée.

Le cadre 17 présente donc une partie interne évidée susceptible de laisser passer le rayonnement gamma. Pour éviter de polluer l'intérieur du détecteur par de la poussière, ou plus simplement pour faciliter la manipulation du cadre 17, celui-ci peut être muni dans son intérieur d'une plaque, de plastique transparent. Compte-tenu de ce qu'on cherche à utiliser des machines standard, on réalise de préférence des cadres 17 dont le poids avoisine le plus possible le poids du collimateur 18 qu'ils sont censés remplacer. Ce faisant, le mouvement de rotation de la gamma caméra 1 autour de l'axe 7 n'est pas perturbé.

Le fait que le bras s'engage dans les coulisseaux confère à la gamma caméra une grande universalité sans le bras la gamma caméra redevient une machine classique.

La figure 2 montre une seringue 22, par exemple en verre ou en plastique. Celle-ci comporte un corps 23 et un piston 24. Le corps 23 est muni d'un orifice 25. Dans un exemple, l'orifice 25 comporte une excroissance munie d'un filet 26. L'excroissance 25 peut ainsi- être vissée dans la partie correspondante d'une tige 27 creuse qui plonge dans un bocal 28. Le bocal 28 est de préférence en plomb. Il peut être opaque. La tige 27 est maintenue dans le bocal 28 par un bouchon 29. Le bocal contient une dilution de produit radioactif : de préférence le même que celui qui est injectable dans le patient. Compte-tenu de la capillarité des liquides devant être transportés depuis le bocal dans la seringue 22, les diamètres de la tige 27 et de l'orifice 25 sont tels qu'ils s'opposent naturellement à l'écoulement du fluide 30 contenu dans le flacon 28.

Le mode de remplissage est le suivant. Le flacon est posé normalement sur son fond 31, la seringue 22 est vissée sur la tige 27, et le piston 24 est remonté de manière à ce que la seringue se remplisse. Une fois que le remplissage est effectué, l'ensemble bocal 28 seringue 22 est alors retourné de manière à ce que le bocal soit maintenant placé au-dessus de la seringue. Ce faisant, des bulles d'air 32 qui subsisteraient dans la seringue 22, viennent se placer près de l'orifice 25 : elles sont expulsées les premières dès que l'on repousse le piston. On repousse alors le piston 24 jusqu'à ce qu'il occupe une position prédéterminée 33 par rapport au corps 23. Ce faisant, on assure d'une part que la quantité de fluide 30 dans la seringue est toujours la même et, d'autre part, que les bulles d'air ont disparu. Ensuite on mesure l'activité de la source dans un activimètre et on ajuste le volume de liquide dans la seringue, en réitérant les mêmes opérations, de façon à obtenir l'activité désirée.

Ceci est important parce que, dans l'invention, le patient est immobile sur le panneau 4 et la gamma caméra 1 tourne autour de lui. En conséquence, la source 11 prend des positions verticales soit au-dessus, soit en-dessous du patient. Si on n'y prenait pas garde, la position des bulles telles que 32 fausserait la position du centre de gravité du liquide radioactif contenu dans la seringue et donc la position du point focal d'émission.

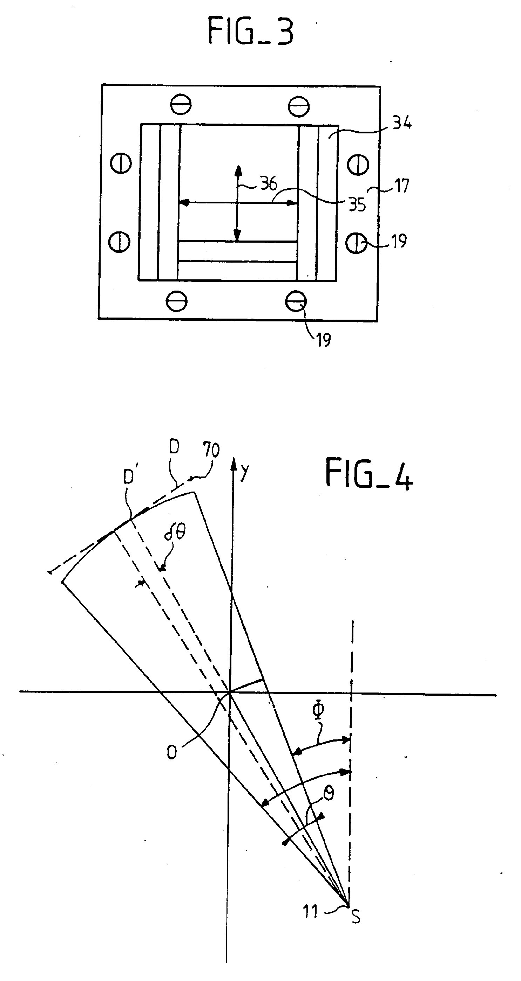

La figure 3 montre un cadre 17 muni de ses dispositifs d'ancrage 19 et d'un diaphragme 34 à volets. Ce diaphragme possède ici deux jeux 35 et 36 de volets permettant de réduire le champ de la gamma caméra vu par la source 11. Ceci permet d'éviter que des régions du détecteur de la caméra, qui ne seraient pas situées dans l'image projetée sur elle du corps à étudier, ne soient saturées par une trop grande quantité d'énergie provenant directement de la source 11. Par exemple si on place la tête du patient devant la gamma caméra, celle-ci ne masquera pas tout le champ de la gamma caméra. Les parties non masquées recevraient autrement un rayonnement direct. D'une manière préférée, le diaphragme n'est pas à déplacement symétrique. Au contraire, les volets, dans au moins une direction, par exemple direction 36 (figures 1 et 3), sont prévus pour laisser une ouverture en bord du plan du détecteur plutôt qu'en son centre. De cette façon, des parties latérales, non hachurées et éloignées du bâti 5 du détecteur D, sont par exemple actives en détection. En conséquence le support 4 peut être déplacé dans le sens de la flèche 60 de façon que seule la tête du patient soit placée sous le détecteur D de la gamma caméra. Le reste du détecteur est alors en porte-à-faux par rapport à la tête. Dans ce cas la potence de la structure 8, qui est télescopique, peut être rentrée dans sa jambe 61 de façon que le détecteur passe le plus près possible de la tête du patient, en pratique à quelques centimètres du nez de celui-ci. Dans ces conditions, le détecteur est alors plus près de la tête que s'il devait contourner le ventre du patient, ventre qui est naturellement plus volumineux. Dans ce même but, le plateau 4 est également moins large, en 62, là où le patient doit poser sa tête que là où reponse son tronc. Ceci conduit à une meilleure résolution du fait que le détecteur peut être placé plus près du corps de ce patient.

Par ailleurs, compte-tenu de la sensibilité plus grande, sans collimateur, de la gamma caméra, on peut rétrécir la fenêtre en énergie et sélectionner les photons détectés. Ceci permet de diminuer les effets du diffusé sans pour autant entraîner une perte d'homogénéité de la gamma caméra. En effet, les photons diffusés ont une énergie plus faible. Les photons diffusés détectés en absence de collimation faussent le résultat de la détection puisque leurs directions et leurs parcours dans le détecteur sont complètement aléatoires et arbitraires. Dans l'invention, comme on a beaucoup de photons, on peut être plus exigeant sur les photons pris en compte et ne considérer que ceux dont l'énergie correspond exactement (avec une plage faible) à l'énergie émise par la source. Dans ce but, on programme avec un bouton de réglage 37 la plage de détection des moyens de traitement 2. Dans l'invention, on réduit la plage de détection à moins de 10 % de la valeur nominale de l'énergie à détecter approximativement à 5 %. On rappelle qu'en détection d'émission classique, la plage est de l'ordre de 20 % de la valeur nominale de l'énergie.

La correction de pondération dont il est question pour tenir compte de la géométrie cônique d'acquisition est due au caractère plan du détecteur. Elle est la suivante. La figure 4 montre en coupe le détecteur D, de centre D', situé à une distance SD de la source 11. Le détecteur est vu de la source 11 sous un angle e constant. Les rayons qui atteignent le détecteur sur son bord latéral droit 70 font, pour une direction d'acquisition donnée, un angle $\Phi$ avec un axe OY d'un repère OXOY. Le repère OXOY passe par le centre de rotation de la gamma caméra. Le centre de rotation est placé sur l'axe 7. L'angle $\Phi$ caractérise la direction de projection. La projection d'un secteur échantillonné $\delta\theta$ sur le détecteur D occupe sur ce détecteur une surface qui dépend de l'écart, par rapport à une direction moyenne SO d'irradiation, de la position de détection. Dans l'invention on corrige par pondération en chaque endroit du détecteur la mesure effectuée par un coefficient qui tient compte de l'atténuation provoquée par cette occupation de surface. L'inverse de coefficient peut s'écrire, pour la projection d'un secteur $S_1$ contigü à la direction moyenne SO,

$$a_1 = SD.tg\ \delta\theta$$

Pour un secteur suivant, elle s'écrit :

$$a_2 = SD.(tg\ 2\delta\theta \cdot tg\ \delta\theta).$$

Pour un détecteur correspondant à un secteur encore plus décalé, elle s'écrit :

$$a_i = SD.(tg\ i\delta\theta - tg(i - 1)\delta\theta).$$

On corrige les valeurs mesurées en fonction de ces coefficients. Cette manière de faire permet d'utiliser un algorithme connu de reconstruction alors que la proximité de la source combinée au caractère plan du détecteur s'y opposaient. Ce faisant on utilise des algorithmes classiques de reconstruction (pas de développement spécifique) et surtout on peut approcher la source 11 ce qui rend le dispositif maniable alors qu'il ne peut pas l'être si la source doit être loin.

On notera que la correction de pondération peut être effectuée en tenant compte de l'écart par rapport à la direction moyenne mesurée selon une direction (35) et aussi selon l'autre (36). Avec l'écart mesuré selon une seule direction la correction est déjà suffisante pour supprimer un effet de "cuvette" dans la reconstruction des coefficients d'atténuation en transmission.

En ce qui concerne la reconstruction proprement dite des coefficients d'atténuation sur la base des valeurs de détection mesurées et corrigées, on met en oeuvre soit des algorithmes de reconstruction de type parallèle avec réarrangement des projections, soit des algorithmes de reconstruction de type faisceau plat en éventail sans réarrangement. Par exemple pour ces derniers, l'algorithme pourra être du type décrit par p. LEWITT dans "Computerized tomography with fan beam geometry", Journal of Computed Assisted Tomography, 1 (4) 1977, ou encore de celui décrit par S. WEBB, SUTCLIFFE, BURKINSHAW, HORSMAN : "Tomography EC reconstruction from exponentially obtained fan beam projection" IEEE transactions on Medical Imaging Vol MI-6, N° 1, mars 1987. On effectue aussi sur les valeurs mesurées une log normalisation de type connu. Cette log normalisation concerne plutôt que les coefficients eux-mêmes la manipulation des logarithmes des coefficients d'atténuation. Ceci simplifie les calculs. Cette log normalisation est connue par ailleurs.

On néglige par ailleurs le fait que l'irradiation est totalement cônique pour l'assimiler à une irradiation en part de galette. La pondération effectuée est suffisante pour ne pas être géné par cette approximation.

Avec la gamma caméra de l'invention, on est capable de réaliser en une minute une soixantaine de vues sur un demi-tour. Cette soixantaine de vues permet de déterminer le coefficient d'absorption du rayonnement gamma à l'intérieur du corps dans des éléments de volume dont la dimension est de l'ordre de 5 millimètres de côté. Une fois que ces coefficients d'absorption ont été acquis, on corrige, selon des techniques connues, des images de tomographie obtenues par émission et dans lesquelles on avait considéré que l'atténuation de transmission était nulle. Un tel mode de correction a par exemple été décrit dans le premier article cité ci-dessus.

## Revendications

1.  Dispositif à scintillation comportant une gamma caméra (1), utilisable pour mesurer l'atténuation de transmission, au travers d'un corps à examiner, d'un rayonnement gamma émis par une source (11) ponctuelle radioactive maintenue en correspondance d'un détecteur (D) de ladite gamma caméra, ledit détecteur étant démuni d'un collimateur (18), et étant placé d'un premier côté du corps, opposé par rapport à un autre côté du corps où est placée la source ponctuelle, et l'ensemble formé par ladite source ponctuelle et ledit détecteur étant dépaçable en un mouvement de rotation relatif par rapport au corps, caractérisé en ce que ladite gamma caméra est montée sur une structure mobile ( 8), cette structure étant susceptible de faire tourner la gamma caméra dans un plan vertical autour du corps disposé en position horizontale allongé sur un support (4), en ce que cette structure entraîne également la source ponctuelle de rayonnement gamma, et en ce que des moyens sont prévus pour pondérer l'atténuation mesurée en fonction de l'éloignement source détecteur et en fonction du caractère plan du détecteur.

2.  Dispositif selon la revendication 1, caractérisé en ce que le détecteur de la gamma caméra est muni d'un cadre amovible placé sur le pourtour du détecteur.

3.  Dispositif selon la revendication 2, caractérisé en ce que le cadre comporte un diaphragme (34) pour réduire le champ de la gamma caméra vu par la source ponctuelle.

4.  Dispositif selon la revendication 2 ou la revendication 3, caractérisé en ce que le cadre a un poids sensiblement égal au poids du collimateur absent.

5. Dispositif selon la revendication 3, caractérisé en ce que le diaphragme possède des volets à déplacement dissymétrique.

6. Dispositif selon l'une quelconque des revendications 1 à 5, caractérisé en ce que la source ponctuelle est contenue dans une seringue.

7. Dispositif selon la revendication 6, caractérisé en ce qu'il comporte un bocal (28) muni d'une tige creuse (27) plongeant par une extrémité à l'intérieur du bocal, et pourvue à son autre extrémité d'un réceptacle pour recevoir un orifice (25) de la seringue.

8. Dispositif selon l'une quelconque des revendications 1 à 7, caractérisé en ce qu'il comporte dans des moyens de traitement (2) du signal détecté des moyens (37) pour réduire la plage d'énergie détectable à moins de 10 % de la valeur nominale de l'énergie des photons gamma à détecter.

## Patentansprüche

1. Szintillationsvorrichtung mit einer Gamma-Kamera (1) zur Messung der Transmissionsabschwächung , quer durch einen zu untersuchenden Körper, einer Gammastrahlung, die von einer radioaktiven Punktquelle (11) abgegeben wird, die gegenüber einem Detektor (D) der Gamma-Kamera gehalten wird, wobei der Detektor von einem Kollimator (18) befreit und auf einer ersten Seite des Körpers gegenüber einer weiteren Seite des Körpers angeordnet ist, auf der die Punktquelle angeordnet ist, und wobei die durch die Punktquelle und den Detektor gebildete Einheit gegenüber dem Körper drehbar ist, dadurch gekennzeichnet, daß die Gamma-Kamera an einem beweglichen Aufbau (8) montiert ist, durch den die Gamma-Kamera in einer Vertikalebene um den Körper drehbar ist, der horizontal ausgestreckt auf einem Träger (4) liegt, daß dieser Aufbau auch die Gammastrahlungs-Punktquelle mitbewegt, und daß Mittel vorgesehen sind, um die gemessene Abschwächung in Abhängigkeit von dem Abstand Quelle-Detektor und in Abhängigkeit von der Planheit des Detektors zu gewichten.

2. Vorrichtung nach Anspruch 1, dadurch gekennzeichnet, daß der Detektor der Gamma-Kamera mit einem beweglichen Rahmen versehen ist, der an dem Außenumfang des Detektors angeordnet ist.

3. Vorrichtung nach Anspruch 2, dadurch gekennzeichnet, daß der Rahmen eine Blende (34) enthält, um das von der Punktquelle gesehene Feld der Gamma-Kamera zu verringern.

4. Vorrichtung nach Anspruch 2 oder 3, dadurch gekennzeichnet, daß der Rahmen ein Gewicht besitzt, das im wesentlichen gleich dem Gewicht des fehlenden Kollimators ist.

5. Vorrichtung nach Anspruch 3, dadurch gekennzeichnet, daß die Blende Verschlußklappen mit unsymmetrischer Verstellung aufweist.

6. Vorrichtung nach einem der Ansprüche 1 bis 5, dadurch gekennzeichnet, daß die Punktquelle in einer Spritze enthalten ist.

7. Vorrichtung nach Anspruch 6, dadurch gekennzeichnet, daß sie einen Glasbehälter enthält, der mit einer Hohlstange (27) versehen ist, die mit einem Ende in das Innere des Glasbehälters eintaucht und an ihrem anderen Ende einen Sammelbereich zur Aufnahme einer Öffnung (25) der Spritze aufweist.

8. Vorrichtung nach einem der Ansprüche 1 bis 7, dadurch gekennzeichnet, daß sie unter den Mitteln (2) zur Verarbeitung des erfaßten Signals Mittel (37) enthält, um den erfaßten Energiebereich auf weniger als 10% des Nennwertes der zu erfassenden Energie der Gamma-Photonen zu verringern.

## Claims

1. Scintillation device comprising a gamma camera (1), usable for measuring transmission attenuation, through a body to be examined, of a gamma ray emitted by a radioactive point source (11) held in position relative to a detector (D) of the said gamma camera, the said detector being without a collimator (18) and being positioned on one side of the body, opposite another side of the body where the point source is positioned, the assembly formed by the said point source and the said detector being displaceable in a rotary movement in relation to the body, characterised by the fact that the said gamma camera is mounted on a mobile structure (8), this structure being capable of causing the gamma camera to turn in a vertical plane about the body positioned horizontally and extending on a support (4), that this structure also entrains the point source of the gamma ray and that means are provided for weighting the measured attenuation in accordance with the distance between the source and the detector and in accordance with the flat nature of the detector.

2. Device in accordance with claim 1, characterised by the fact that the detector of the gamma camera is fitted with a movable frame positioned on the periphery of the detector.

3. Device in accordance with claim 2, characterised by the fact that the frame comprises a diaphragm (34) serving to reduce that field of the gamma camera which is seen by the point source.

4. Device in accordance with claim 2 or claim 3, characterised by the fact that the weight of the frame is approximately equal to that of the absent collimator.

5. Device in accordance with claim 3, characterised by the fact that the diaphragm has asymmetrically moving shutters.

6. Device in accordance with any one of claims 1 to 5, characterised by the fact that the point source is contained in a syringe.

7. Device in accordance with claim 6, characterised by the fact that it comprises a flask (28) fitted with a hollow rod (27) immersed by one end in the flask and provided at its other end with a receptacle to accommodate an orifice (25) of the syringe.

8. Device in accordance with any one of claims 1 to 7, characterised by the fact that it comprises, in the means for processing (2) the signal detected, means (37) for reducing the energy threshold detectable to less than 10% of the nominal value of the energy of the gamma photons to be detected.

FIG_1

FIG_2

EP 0 484 430 B1

# FIG_3

# FIG_4